# EUROPEAN PATENT SPECIFICATION

(11) **EP 1 673 434 B1**
(45) Date of publication and mention of the grant of the patent: **16.09.2009**
(21) Application number: 04768901.3
(22) Date of filing: 14.10.2004
(51) Int. Cl.: C12M 1/00, B25J 21/02, B01L 1/02

(54) **LABORATORY APPARATUS WITH TWO CABINETS**
LABORVORRICHTUNG MIT ZWEI SCHRANKEINHEITEN
APPAREIL DE LABORATOIRE AVEC DEUX ENCEINTES

(30) Priority: 15.10.2003 GB 0324110
(43) Date of publication of application: 28.06.2006
(73) Proprietor: Ruskinn Life Sciences Limited, Pencoed Bridgend CF35 5HZ (GB)
(72) Inventor: MURRAY, Fergus, Francis, Shipley BD18 1JJ (GB); SKINN, Andrew, Faweather Grange, High Eldwick BD16 3BL (GB)
(74) Representative: Davies, Gregory Mark
(86) International application number: PCT/GB2004/004371
(87) International publication number: WO 2005/040330

(56) References cited:
- FR-A- 2 605 016
- US-A- 2 244 082
- US-A- 4 111 753
- US-A- 4 262 091
- US-A- 5 169 217
- US-A- 5 861 305

## Description

This invention relates to a laboratory apparatus and more particularly but not solely to a laboratory apparatus for use in the In Vitro Fertilisation treatment of infertility.

Over the course of the last 25 years or so, infertile couples have been able to take advantage of an In Vitro Fertilisation or so-called IVF technique to improve their chances of reproduction, whereby the female is treated with hormones such that a large number of unfertilised eggs or so-called oocytes are produced. The oocytes are extracted from the patient and taken to a laboratory usually situated in the next room, where they are washed and counted.

It is desirable to collect a large number of oocytes so that the chances of a successful embryo being produced are increased. Accordingly, the above process is repeated several times until a suitable number of oocytes have been collected.

The oocytes are then fertilised, either by the introduction of spermatozoa into the dish containing oocytes or by using micromanipulators to inject spermatozoa into individual oocytes. The fertilised oocytes or so-called Zygotes are then transferred to an incubator to an environment which is controlled to closely resemble the conditions inside the body. Typically, the carbon dioxide level inside the body is constant at 5%, thereby keeping the pH value constant. Also, the temperature is substantially constant at 37°C.

The Zygotes are kept inside the incubator for a period of at least several days until the embryo stage is reached. As hereinbefore described, a plurality of oocytes are collected and fertilised and accordingly, it is possible that several manage to reach the embryonic stage. At this point, one or more of the best embryos are selected and implanted into the female, although it will be appreciated that the implantation of multiple embryos could result in a multiple pregnancy.

Unfortunately, the success rate of the above-mentioned IVF procedure is low and many couples have to undergo the procedure several times before pregnancy is achieved. It will be appreciated that this is both expensive and distressing.

Following a detailed study of the above mentioned IVF procedure, we have realised that a high proportion of the failures are due to the effects of the environment in which the culture is kept at the various stages following the extraction of the oocyte from the female and prior to implanting the cultured embryo into the female uterus.

The first problem is that, following extraction from the female the oocytes are exposed to the normal room environment whilst they are washed and counted. This problem is exacerbated by the fact that a delay of several minutes or so is incurred each time the embryologist has to wait for further oocytes to be collected from the female.

The second problem is that, once inside the incubator, the controlled environment therein is regularly disturbed each time the incubator is opened to add or remove cultures. It will be appreciated that the incubator contains cultures from many couples which are continuously being added and removed. Each time the incubator is opened, the environment therein mixes with the room environment and it can take a considerable time to recover the ideal environment once the door is closed. US4111753 disuses apparatus where the environment can be controlled using a closed system with hand access ports but being a closed system, there is no means for ease of access to the system by having a cabinet with an open front.

Another problem is that each culture has to be regularly examined, which necessitates removal of the culture from the incubator and inspection, for example under a microscope, prior to replacement in the incubator.

This process of inspection further disturbs the environment within the incubator and exposes the culture to the room environment whilst the inspection is carried out.

We have now devised a laboratory apparatus particularly suited for use in the IVF process, which alleviates the above mentioned problems.

In accordance with this invention, there is provided a laboratory apparatus as set out in claim 1.

In use, following extraction of the oocytes, they are taken to the first cabinet where they can conveniently be washed and counted through the open front, which affords ready access into and out of the cabinet. The air within the first cabinet is circulated and thus the oocytes are contained in a substantially constant environment. The oocytes are contained in this constant environment until a sufficient number have been collected. The oocytes are then mixed with the spermatozoa before being transferred into the second cabinet through the passageway, by opening the closure there between.

The closure serves to contain air within the second cabinet, although any air which does pass into the second cabinet during transfer will merely be air from the first cabinet and not that from the room environment.

Once inside the second cabinet, the cultures are kept in an environment in which the gas and temperatures levels are controlled, preferably to substantially resemble the conditions. that would be present inside the body.

Any cultures which are added or removed from the second cabinet have to pass through the passageway via the first cabinet and thus the environment inside the second cabinet is not directly exposed to the room environment.

The inspection means enables the cultures in the second cabinet to be inspected, thereby avoiding having to regularly remove them from the cabinet.

The present invention therefore retains the cultures in a stable and controlled environment substantially all of the time from extraction of the oocytes to implantation of the embryo. In this manner the success rate of the IVF procedure is greatly increased over the traditional procedure.

Preferably the circulated air inside the first cabinet is passed through a filter in order to alleviate risk of contamination of the culture by airborne particles.

Preferably the airflow inside the first cabinet is a laminar flow, which preferably extends downwards, parallel to said open front of the cabinet. This laminar flow helps to constrain the air within the cabinet.

Preferably means are provided for controlling the temperature inside the first cabinet. In order to conserve energy and avoid the need to heat the air inside the first cabinet, the heating means is preferably arranged to heat a surface inside the first cabinet on which containers containing matter such as the oocytes can be placed.

Preferably the means for closing the passageway comprises first and second closures which are spaced apart on the passageway with a space there between. Thus, in order to transfer the cultures between the first and second cabinets, they have to pass through two closures which further serve to isolate the environment than the second cabinet, by opening one closure, placing the cultures in the space between the two closures and closing the open closure before opening the second closure.

Preferably means are provided for changing the air in the space between the two closures, so that the environment therein is as close as possible to that in the second cabinet before the closure isolating the space and the second cabinet is opened. This further serves to minimise any disruption to the environment inside the second cabinet when cultures are added or moved.

Preferably the closures are interlocked to provide a time delay between opening of the closures.

Preferably means are provided for transporting the cultures between said first and second cabinets through the passageway.

Preferably said means for controlling the level of at least one gas within the cabinet is arranged to control the level of carbon dioxide and/or oxygen within the cabinet.

Preferably means are provided for controlling the humidity inside the second cabinet.

Preferably the pressure inside the second cabinet is greater than atmospheric pressure to help prevent the ingress from the room into the cabinet.

Preferably the second cabinet comprises a transparent front wall, through which the cultures can be inspected.

A microscope or a camera may be provided inside the second cabinet to further enable inspection of cultures therein.

Preferably means are provided to enable manipulation of the cultures in the second cabinet. Preferably said means enabling manipulation comprises at least one port in the front wall of the second cabinet, through which a person's hand can sealingly extend.

An embodiment of this invention will now be described by way of an example only and with reference to the accompanying drawings, in which:
Figure 1 is a front view of a laboratory apparatus in accordance with this invention for use in the IVF procedure; and
Figure 2 is a sectional view along the line I-II of Figure 1.

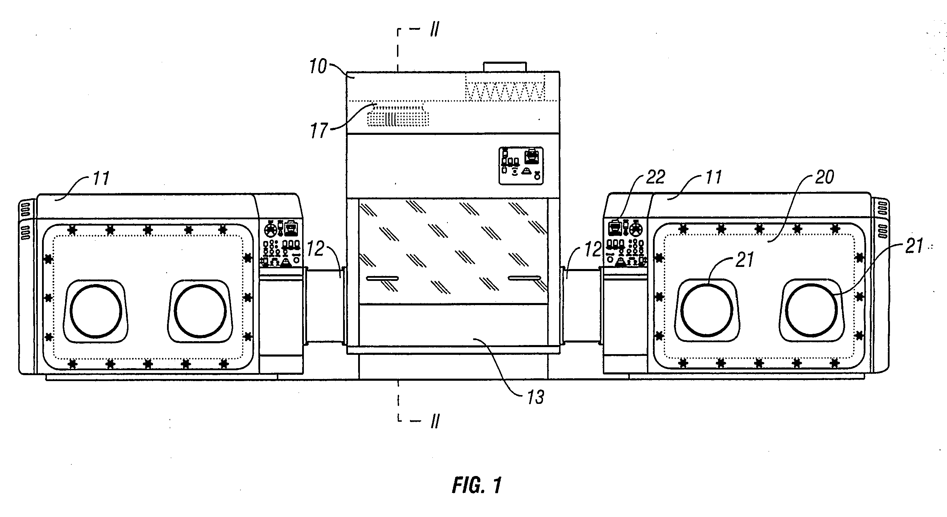

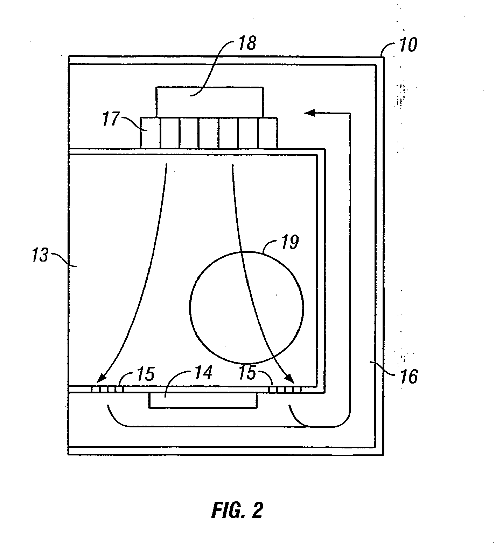

Referring to Figures 1 and 2 of the drawings, there is shown a laboratory apparatus for use in the IVF procedure comprising a first central cabinet 10 disposed between two identical outer cabinets 11. Each of the outer cabinets 11 is connected to the central cabinet by a respective duct 12, which respectively provide a sealed passageway between the interiors of the respective cabinets 10, 11. The central cabinet 10 comprises an open front wall 13 which allows easy access to the interior of the cabinet 10. A heater 14 is disposed under the floor of the interior of the cabinet 10. Apertures 15 are formed in the floor of the interior of the cabinet 10 to provide communication between the interior of the cabinet and a duct 16 which extends under the floor and behind the rear wall of the interior cabinet to a cavity formed above the top wall of the interior of the cabinet. A fan 17 is arranged to draw air from the cavity through a hepa filter 18 to create a downward laminar flow inside the cabinet 10. The air is then drawn through the apertures 15 in the floor and recirculated.

The cabinet 10 may be arranged to draw a percentage of filtered air from the environment to compensate for losses and to help maintain the air quality inside the air cabinet.

The heater 14 is connected to a temperature control circuit arranged to maintain a constant temperature of 37°C on the floor of the interior of the cabinet 10.

One end of each duct 12 is connected to an aperture 19 formed in respective side walls of the cabinet 10 and closures (not shown) are provided at opposite ends of the ducts 12 to close the passageways therethrough.

The opposite end of each duct 12 is connected to a corresponding aperture in the side wall of the respective outer cabinet 11. Each outer cabinet 11 comprises a sealed interior compartment and a transparent front wall 20 which allows the interior of the compartment to be viewed. A pair of ports 21 are provided in the front wall 20 of the cabinet 11, through which a person is able to insert their respective arms into the interior of the cabinet 11. The ports 21 may be provided with gloves or sleeves which serve to provide isolation between the interior and exterior of the cabinet 11.

A plurality of controls 22 are provided on the cabinet 11 for controlling the temperature, humidity and pH levels inside the cabinet 11. The pH of the cultures inside the cabinet 11 may be controlled by altering the levels of gases, such as carbon dioxide, within the cabinet. The level of oxygen may also be controlled, so that the gas concentrations within the cabinet 11 resemble that inside the womb. Monitors and alarms may be provided for monitoring the respective levels inside the cabinet.

A microscope (not shown) may also be provided to enable cultures within the cabinet 11 to be examined.

In use, the oocytes collected from the female patient are transferred directly on a dish into the open-fronted cabinet 10, where the dish is placed on the floor of the interior cabinet above the heater 14, which maintains the temperature of the oocytes at 37°C (i.e. at a temperature which is substantially similar to that inside the female body). The oocytes are then washed and counted and, if necessary, the oocytes are kept inside the cabinet 10 until a sufficient number have been collected. This whole process may take 15 minutes or so but it will be appreciated that the laminar airflow inside the cabinet 10 is clean and serves to contain the oocytes in a stable environment which is substantially isolated from the room environment.

Once a sufficient number of oocytes has been collected, they are mixed with spermatozoa prior to transfer into one of the outer cabinets 11.

In order to transfer the cultures into one of the outer cabinets 11, the door at the inner end of the passageway connecting the cabinets 10, 11 is opened and the cultures are then placed in the duct 12 before the door is closed. The air inside the sealed duct is then brought up to the same environmental conditions as that inside the outer cabinet 11 before the door from the duct 12 to the outer cabinet 11 can be opened.

The cultures are then kept in the outer cabinet 11 in an environment having a stable temperature, pH and humidity, closely resembling the conditions inside the human body. The fertilised oocytes and the resultant embryos can readily be inspected through the transparent front wall 20 of the cabinet 11 without the need to remove them from the cabinet, thereby avoiding disturbance of the environment therein.

A microscope or other inspection tool may also be provided to facilitate inspection of the cultures inside the cabinet 11. The ports 21 in the front wall of the cabinet 11 also enable the cultures to be manipulated by hand.

Once an embryo is ready for implantation, it can be removed from the cabinet via the duct 12 in the reverse manner to that as hereinbefore described. In this manner, any cultures remaining inside the cabinet 11 are not disturbed while the cultures are removed.

An apparatus in accordance with this invention is relatively simple in construction yet substantially improves the success rate of the IVF procedure.

## Claims

1. A laboratory apparatus comprising first (10) and second cabinets (11) interconnected by a passageway (12) and closing means for closing said passageway, said first cabinet comprising an open front (13) and means for circulating air within said first cabinet, (10) said second cabinet (11) being substantially sealed and having means for controlling the temperature within said second cabinet, means for controlling the level of at least one gas within said second cabinet and means to allow external inspection of the contents of said second cabinet, wherein the closing means for closing the passageway (12) comprises first and second closures which are spaced apart on the passageway (12) with a space there between and said first and second closures are arranged such that a first closure which has opened is closed before a second closure can be opened,
**characterised in that** there also being means for causing the environmental conditions inside the closed passageway (12) to be brought up to the same environmental conditions as that inside the second cabinet (11) before said second closure can be opened.

2. A laboratory apparatus as claimed in Claim 1, in which said circulated air inside said first cabinet (10) is passed through a filter in order to alleviate risk of contamination of the culture by airborne particles.

3. A laboratory apparatus as claimed in Claims 1 or 2, in which the circulated air inside the first cabinet forms (10) an airflow, the airflow being a laminar flow which extends downwardly, parallel to said open front of said first cabinet.

4. A laboratory apparatus as claimed in any preceding claim, in which means are provided for controlling the temperature inside said first and second cabinets (10, 11).

5. A laboratory apparatus as claimed in any preceding claim, in which said closures are interlocked to provide a time delay between opening of the closures.

6. A laboratory apparatus as claimed in any preceding claim, in which means are provided for changing the air in the space between the two closures.

7. A laboratory apparatus as claimed in any preceding claim, in which means are provided for transporting the cultures between said first and second cabinets (10,11) through the passageway (12).

8. A laboratory apparatus as claimed in any preceding claim, in which said means for controlling the level of at least one gas within the second cabinet (11) is arranged to control the level of carbon dioxide and/or oxygen within the second cabinet (11).

9. A laboratory apparatus as claimed in any preceding claim, in which means are provided for controlling the humidity inside said second cabinet (11).

10. A laboratory apparatus as claimed in any preceding claim, in which means are provided for creating a pressure inside said second cabinet (11) which is greater than atmospheric pressure.

11. A laboratory apparatus as claimed in any preceding claim, in which said second cabinet (11) comprises a transparent front wall (20) through which the cultures can be inspected.

12. A laboratory apparatus as claimed in any preceding claim, in which means (21) are provided to enable manipulation of the cultures in said second cabinet (11).

## Patentansprüche

1. Laborgerät aufweisend eine erste (10) und eine zweite Kammer (11), die über einen Durchgang (12) verbunden sind, und Verschlußmittel zum Verschließen des Durchganges, wobei die erste Kammer eine offene Front (12) und Mittel zum Umwälzen von Luft in der ersten Kammer (10) aufweist, die zweite Kammer (11) im wesentlichen abgeschlossen ist und Mittel zum Steuern der Temperatur in der ersten Kammer hat, und Mittel zum Steuern des Pegels mindestens eines Gases in der zweiten Kammer und Mittel zum Ermöglichen einer externen Kontrolle von Inhalten der zweiten Kammer vorgesehen sind, wobei die Verschlußmittel zum Verschließen des Durchgangs (12) erste und zweite Verschlüsse aufweisen, welche im Durchgang (12) mit einem Abstand zueinander beabstandet sind und welche so angeordnet sind, daß ein offener erster Verschluß geschlossen wird, bevor ein zweiter Verschluß geöffnet werden kann **dadurch gekennzeichnet, daß** weiter Mittel vorhanden sind, um die Umweltbedingungen im verschlossenen Durchgang (12) an die in der zweiten Kammer (11) anzugleichen, bevor der zweite Verschluß geöffnet werden kann.

2. Laborgerät gemäß Anspruch 1, bei dem die in der ersten Kammer (10) umgewälzte Luft durch einen Filter geführt wird, um das Risiko von Kontaminationen von Kulturen durch in der Luft befindliche Teilchen zu vermindern.

3. Laborgerät gemäß Anspruch 1 oder 2, bei dem die in der ersten Kammer (10) umgewälzte Luft einen Luftstrom bildet, welcher laminar ist und parallel zu der offenen Front der ersten Kammer nach unten verläuft.

4. Laborgerät gemäß einem der obigen Ansprüche, bei dem Mittel zum Steuern der Temperatur in der ersten und zweiten Kammer (10, 11) vorgesehen sind.

5. Laborgerät gemäß einem der obigen Ansprüche, bei dem die Verschlüsse so verriegelt sind, daß eine Zeitverzögerung zwischen dem Öffnen der Verschlüsse vorgesehen ist.

6. Laborgerät gemäß einem der obigen Ansprüche, bei dem Mittel zum Wechseln der Luft im Raum zwischen den beiden Verschlüssen vorgesehen sind.

7. Laborgerät gemäß einem der obigen Ansprüche, bei dem Mittel zum Transportieren von Kulturen zwischen der ersten und der zweiten Kammer (10, 11) durch den Durchgang (12) vorgesehen sind.

8. Laborgerät gemäß einem der obigen Ansprüche, bei dem die Mittel zum Steuern des Pegels mindestens eines Gases in der zweiten Kammer (11) so ausgebildet sind, daß sie den Pegel von Kohlendioxid und/oder Sauerstoff in der zweiten Kammer (11) steuern.

9. Laborgerät gemäß einem der obigen Ansprüche, bei dem Mittel zum Steuern der Feuchtigkeit in der zweiten Kammer (11) vorgesehen sind.

10. Laborgerät gemäß einem der obigen Ansprüche, bei dem Mittel zum Erzeugen eines überatmosphärischen Drucks in der zweiten Kammer (11) vorgesehen sind.

11. Laborgerät gemäß einem der obigen Ansprüche, bei dem die zweite Kammer (11) eine transparente Vorderwand (20) aufweist, durch welche Kulturen kontrolliert werden können.

12. Laborgerät gemäß einem der obigen Ansprüche, bei dem Mittel (21) zum Ermöglichen einer Manipulation von Kulturen in der zweiten Kammer (11) vorgesehen sind.

## Revendications

1. Appareil de laboratoire avec une première (10) et une seconde (11) enceintes interconnectées par une conduite (12) et des moyens de fermeture pour fermer la conduite, ladite première enceinte comportant une face frontale ouverte (13) et des moyens pour faire circuler l'air à l'intérieur dans ladite première enceinte (10), ladite seconde enceinte (11) étant substantiellement étanche et ayant des moyens pour Controller la température dans ladite seconde enceinte, des moyen pour Controller le niveau d'au moins un gaz dans ladite seconde enceinte et des moyens pour permettre l'inspection depuis l'extérieur du contenu de ladite seconde enceinte, les moyens de fermeture pour fermer la conduite (12) comprenant un premier et un second obturateurs espacés sur la conduite (12) par un intervalle entre eux et lesdits premier et second obturateurs étant disposés de telle façon qu'un premier obturateur à s'ouvrir soit refermé avant que le second ne soit ouvert, **caractérisé en ce qu'**il est aussi prévu des moyens pour porter les conditions environnementales à l'intérieur de la conduite fermée (12) à égalité avec les conditions environnementales à l'intérieur de la seconde enceinte (11) avant que le second obturateur ne soit ouvert.

2. Appareil de laboratoire selon la revendication 1, dans lequel ledit air circulant à l'intérieur de ladite première enceinte (10) passe par un filtre de manière à éliminer le risque de contamination de la culture par des particules transportées par l'air.

3. Appareil de laboratoire selon la revendication 1 ou 2, dans lequel ledit air circulant à l'intérieur de ladite première enceinte (10) forme un flux d'air, celui-ci étant un flux laminaire dirigé vers le bas parallèlement à ladite face frontale ouverte de ladite première enceinte.

4. Appareil de laboratoire selon l'une des revendications précédentes, dans lequel des moyens pour contrôler la température à l'intérieur desdites première et une seconde (10, 11) enceintes sont prévus.

5. Appareil de laboratoire selon l'une des revendications précédentes, dans lequel lesdits obturateurs sont interconnectés afin de ménager un délai entre l'ouverture et la fermeture des obturateurs.

6. Appareil de laboratoire selon l'une des revendications précédentes, dans lequel des moyens sont prévus pour changer l'air dans l'intervalle entre les deux fermetures

7. Appareil de laboratoire selon l'une des revendications précédentes, dans lequel des moyens sont prévus pour transporter les cultures entre lesdites première et une seconde (10, 11) enceintes par ladite conduite.

8. Appareil de laboratoire selon l'une des revendications précédentes, dans lequel le niveau d'au moins un gaz à l'intérieur de la seconde enceinte (11) est utilisé pour contrôler le niveau du dioxyde de carbone et/ou l'oxygène à l'intérieur de la seconde enceinte (11).

9. Appareil de laboratoire selon l'une des revendications précédentes, dans lequel des moyens sont prévus pour contrôler l'humidité à l'intérieur de la seconde enceinte (11).

10. Appareil de laboratoire selon l'une des revendications précédentes, dans lequel des moyens sont prévus pour créer une pression supérieure à la pression atmosphérique à l'intérieur de ladite seconde enceinte (11).

11. Appareil de laboratoire selon l'une des revendications précédentes, dans lequel ladite seconde enceinte (11) comprend une paroi frontale transparente (20) à travers laquelle on peut contrôler les cultures.

12. Appareil de laboratoire selon l'une des revendications précédentes, dans lequel des moyens (21) sont prévus pour permettre la manipulation des cultures à l'intérieur du second cabinet (11).
